# EUROPEAN PATENT SPECIFICATION

(11) **EP 1 695 963 B1**
(45) Date of publication and mention of the grant of the patent: **20.02.2008**
(21) Application number: 05020713.3
(22) Date of filing: 22.09.2005
(51) Int. Cl.: C07D 213/89, A01N 33/16, A01N 55/02

(54) **Pyrithione metal salts having defined particle size distribution and paint composition containing the same**
Metallsalze von Pyrithion mit definierter Korngrössenverteilung und diese enthaltende Färbemittel
Sels metalliques de la pyrithione et compositions tinctoriales les contenant

(30) Priority: 22.02.2005 KR 2005014330
(43) Date of publication of application: 30.08.2006
(73) Proprietor: KOLON INDUSTRIES, INC., Kwacheon-city, Kyunggi-do 427-040 (KR)
(72) Inventor: Park, Chan-Pil, Yongin-si Gyeonggi-do 449-912 (KR); Park, Hee-Jung, Yongin-si Gyeonggi-do 449-771 (KR)
(74) Representative: Merkle, Gebhard

(56) References cited:
- EP-A- 1 080 640
- WO-A-20/04060062

## Description

### Technical Field

The present invention relates to a pyrithione metal salts and a paint composition containing the same. More specifically, the present invention relates to a pyrithione metal salts controlled to have a defined particle size distribution and an anti-fouling paint composition containing the pyrithione metal salts to enhance anti-fouling durability.

### Background Art

Bacteria, protozoa and invertebrates devouringly stick to all the surface of the marine environment, which process is referred to as "attachment" throughout this specification.

The prevention of attachment is of particular interest to those involved in marine transport and ocean engineering, such as beach construction, heat exchanger, marine sensor, underwater implant, aqua farm construction, or the like.

For example, attachment on vessel hulls increases frictional attraction to reduce the corresponding speed and mobility with a rise of fuel consumption and results in an increased maintenance cost for removal of attachment. Moreover, the attachment of only a small number of living organisms on the propeller of a vessel pronouncedly reduces the efficiency of the propeller or causes the corrosion effect.

The significant assemblage of marine life involved in the attachment is living organisms that belong to crustaceans generally called "cirripedia". Among the crustaceans, living organisms called "barnacle" participate in the attachment on the immersed surface such as vessel hulls.

In an attempt to solve this problem, there have been used an antifouling paint, of which the effective components are mostly tin compounds, tributyl tin, or triphenyl tin.

The tin compounds, however, cause accumulation of toxic substances in living bodies to create disorderliness in the marine ecosystem and their use is currently banned in many countries. For that reason, there have been developed low-toxic underwater antifouling agents safe to human body or fish, and antifouling paint formulations of different compositions containing a pyrithione metal salts or a triphenylborane-amine complex to have enhanced performances of antifouling efficiency and antifouling durability.

A known method for preparing an insoluble polyfunctional pyrithione metal salts is disclosed in U.S. Patent No. 2,809,971. Similar compounds and other techniques for preparing those compounds are described in U.S. Patent Nos. 2,786,847; 3,589,999; 3,590,035 and 3,773,770. The pyrithione metal salts prepared by these methods have an average crystallite in the micrometer level and can be processed to have an average particle size of 0.5 to 15 µm through different mechanical treatments (e.g., grinding or milling).

The conventional techniques provide little information about the form and size of pyrithione metal salts used for antifouling paint formulations, so in many cases, there occurs a change in the elementary properties of the paint formulations having the same content of the pyrithione metal salts, such as antifouling durability, film formation condition, oil absorbency, dispersability, etc., on which many studies are still on demand.

The technique that enhances the properties by controlling the pyrithione particle size is disclosed in U.S. Patent Publication 2004-0191331 A1, where in an antidandruff composition containing an granulated zinc material, the granulated zinc material has a particle size of less than 600Å and 90 % of its particles have a particle size of less than 50 µm. The composition of the granulated zinc material having such a defined particle size remarkably enhances the antidandruff effect. However, the only use purpose of this composition is shampoo formulations, and there has been no study on the film formation condition or antifouling durability of the composition in use for antifouling paint formulations.

EP-A-1 080 640 relates to a coated bis(2-pyridinethiol 1-oxide) copper salt which has been coated with one or more members selected among glycerol, dialkyl phthalates, lubricating oils, acrylic resins, rosins, fatty acid amides, di-alkylpolysulfides, polybutene, paraffins, and vaseline.

WO 2004/060062 A1 relates to a composition comprising a non-dusting copper pyrithione dispersion comprising small solid particles of copper pyrithione dispersed in a liquid dispersant, said solid particles having a particle size within a range of from 0.1 to about 10 microns and a median particle size of less than 3 microns.

In an attempt to study the pyrithione metal salts of which the oil absorbency changes even with a defined average particle size and the paint formulations containing the pyrithione metal salts of which the properties such as antifouling durability and film formation condition change, the inventors of the present invention have found out that the pyrithione metal salts having a controlled particle size distribution exhibits a defined range of oil absorbency, that the paint composition containing the pyrithione metal salts has its dispersability remarkably enhanced, and that the antifouling paint using the paint composition is excellent in antifouling film formation condition with enhanced antifouling durability.

It is therefore an object of the present invention to provide a pyrithione metal salts having a controlled particle size distribution to exhibit oil absorbency adequate for a paint manufacture and a good dispersability.

It is another object of the present invention to provide a paint composition containing the pyrithione metal salts to enhance the antifouling durability and guarantee a good condition for antifouling film formation.

### Disclosure of Invention

To achieve the above objects of the present invention, there is provided a pyrithione metal salts including, in the aspect of a particle size distribution, 10 to 40 wt.% of particles having a particle size of less than 2 µm, and 5 to 20 wt.% of particles having a particle size of greater than 10.0 µm.

There is also provided a paint composition containing the pyrithione metal salts having the above-defined particle size distribution.

The present invention will be described in further detail as follows.

In the present invention, the pyrithione metal salts is represented by the following formula 1 and, more specifically, defined as a compound containing at least one selected from zinc pyrithione, copper pyrithione, magnesium pyrithione, barium pyrithione, or strontium pyrithione:

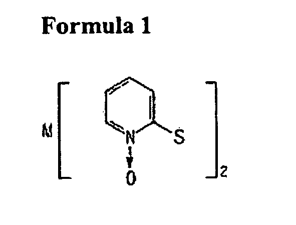

Where M is Zn, Cu, Mg, Ba, or Sr.

The average particle size of the pyrithione metal salts is preferably 3 to 8 µm, more preferably 3.5 to 7 µm. If not specifically limited, this size range is known in regard to the use of pyrithione metal salts as an antifouling effective component.

Even the products having the average particle size exhibit a change in properties as antifouling paint formulations. In the present invention, the content of pyrithione metal salts particles having a defined crystallite size, that is, the particle size distribution of the pyrithione metal salts is controlled to provide a pyrithione metal salts having a defined range of oil absorbency and good dispersability in preparing a paint composition. This is to enhance antifouling durability and film formation condition of the paint composition using the pyrithione metal salts as an effective component.

More specifically, it is desirable to use a pyrithione metal salts having a particle size of less than 2 µm so as to enhance the initial antifouling performance of the antifouling paint. But, the antifouling paint composition including only fine particles of a pyrithione metal salts has a drop of antifouling durability, thereby limiting its use for antifouling paint formulations that are required to maintain the antifouling activity for several years. To overcome this problem, the present invention includes 5 to 20 wt.% of pyrithione metal salts particles having a particle size of greater than 10 µm with respect to the total weight of the pyrithione metal salts.

In consideration of the initial antifouling performance, the present invention includes 10 to 40 wt.% of pyrithione metal salts particles having a particle size of less than 2 µm with respect to the total weight of the pyrithione metal salts. When including less than 10 wt.% of pyrithione metal salts particles having a particle size of less than 2 µm, the paint composition has a low initial antifouling activity. With more than 40 wt.% of pyrithione metal salts particles having a particle size of less than 2 µm, the antifouling durability of the paint composition decreases and the process for preparing a pyrithione metal salts having such a particle size distribution is quite expensive.

For antifouling durability, the present invention includes 5 to 20 wt.% of pyrithione metal salts particles having a particle size of greater than 10 µm. When including less than 5 wt.% of pyrithione metal salts particles having a particle size of greater than 10 µm, the paint composition cannot maintain its antifouling activity for a long-term use. With more than 20 wt.% of pyrithione metal salts particles having a particle size of greater than 10 µm, there are some problems in oil absorbency and dispersability, and the film formation condition is not good when applying the antifouling paint using the pyrithione metal salts of the particle size distribution to a vessel.

The method of preparing a pyrithione metal salts having the average particle size and the particle size distribution includes, if not specifically limited to, various mechanical treatment step, i.e., grinding or milling process after a known process for preparing a pyrithione metal salts.

In using the pyrithione metal salts as an antifouling effective component in the paint composition of the present invention, the content of the pyrithione metal salts is the same as directed in the known preparation method of an antifouling paint composition and, preferably in the range of 1 to 20 wt.% with respect to the total weight of the paint composition.

If not specifically limited, the other components than the pyrithione metal salts as an antifouling effective component, including a resin, pigments, a setting agent, a solvent, and other additives, e.g., conditioner, antifusant, etc. are used according to a known antifouling paint composition.

### Best Mode for Carrying out the Invention

Hereinafter, the present invention will be described in detail by way of the following examples, which are not intended to limit the scope of the present invention.

### <Preparation of Fine Powder of Pyrithione metal salts>

### Example 1: Preparation of Fine Powder of Zinc Pyrithione

1000 g of sodium pyrithione, 20% aqueous solution (1.34 mmol of sodium pyrithione) was added to a 2L reactor equipped with a mechanical agitator and warmed to 70 °C with stirring. To the solution was added 190 g of 50 % zinc chloride (0.7 mmol of zinc chloride) over 10 minutes.

After the completion of the addition, the solution was stirred at 70 °C for one hour, dehydrated and then washed with 500 g of distilled water.

The solid thus obtained was dried with a heated-air drier at 70 °C to have a moisture content of less than 0.5 % and ground with a hammer mill to obtain a find powder of zinc pyrithione.

The zinc pyrithione thus obtained was 212 g with a yield of 99.5 %.

The purity checked by iodometry was 99.2 %. The particle size was measured with a particle size analyzer (Mastersiser 2000 supplied by Malvern Company), the results of which are presented in Table 1.

The oil absorbency of the zinc pyrithione was 0.32 mℓ/g as measured according to KS M ISO 787-5.

The dispersability of the zinc pyrithione was 90 µm as measured according to KS M ISO 1524 and KS M ISO 8780-3, where a smaller value of the measurement indicates better dispersability.

### Example 2: Preparation of Fine Powder of Copper Pyrithione

1000 g of sodium pyrithione, 20% aqueous solution (1.34 mmol of sodium pyrithione) was added to a 2L reactor equipped with a mechanical agitator and warmed to 70 °C with stirring. To the solution was added 186 g of 50 % copper chloride (0.7 mmol of copper chloride) over 60 minutes.

After the completion of the addition, the solution was stirred at 70 °C for one hour, dehydrated and then washed with 500 g of distilled water.

The solid thus obtained was dried with a heated-air drier at 70 °C to have a moisture content of less than 0.5 % and ground with an air jet mill to obtain a find powder of copper pyrithione.

The copper pyrithione thus obtained was 210 g with a yield of 99.3 %.

The purity checked by chemical analysis was 98.7 %. The particle size was measured with a particle size analyzer (Mastersiser 2000 supplied by Malvern Company), the results of which are presented in Table 1.

For this copper pyrithione, oil absorbency and dispersability were 0.35 mℓ/g and 70 µm, respectively, as measured in the same manner as described in Example 1.

### Example 3: Preparation of Fine Powder of Zinc Pyrithione

1000 g of sodium pyrithione, 20% aqueous solution (1.34 mmol of sodium pyrithione) was added to a 2L reactor equipped with a mechanical agitator and warmed to 70 °C with stirring. To the solution was added 190 g of 50 % zinc chloride (0.7 mmol of zinc chloride) over 10 minutes. After the completion of the addition, the solution was stirred at 70 °C for one hour, dehydrated and then washed with 500 g of distilled water.

The solid thus obtained was dried with a heated-air drier at 70 °C to have a moisture content of less than 0.5 %. Two-thirds of the dry solid was ground with a hammer mill and mixed with one-third, which was not ground. The particle size was measured with a particle size analyzer (Mastersiser 2000 supplied by Malvern Company), the results of which are presented in Table 1.

The zinc pyrithione thus obtained was 211 g with a yield of 99.1 %, and the purity checked by chemical analysis was 99.8 %.

For the zinc pyrithione of which two-thirds was ground with a hammer mill and mixed with one-third that was not ground, oil absorbency and dispersability were 0.34 mℓ /g and 100 µm, respectively, as measured in the same manner as described in Example 1.

### Example 4: Preparation of Fine Powder of Copper Pyrithione

1000 g of sodium pyrithione, 20% aqueous solution (1.34 mmol of sodium pyrithione) was added to a 2L reactor equipped with a mechanical agitator and warmed to 70 °C with stirring. To the solution was added 186 g of 50 % copper chloride (0.7 mmol of copper chloride) over 60 minutes. After the completion of the addition, the solution was stirred at 70 °C for one hour, dehydrated and then washed with 500 g of distilled water.

The solid thus obtained was dried with a heated-air drier at 70 °C to have a moisture content of less than 0.5 %. Two-thirds of the dry solid was ground with a hammer mill and mixed with one-third, which was not ground. The particle size was measured with a particle size analyzer (Mastersiser 2000 supplied by Malvern Company), the results of which are presented in Table 1.

The copper pyrithione thus obtained was 210 g with a yield of 99.3 %, and the purity checked by chemical analysis was 98.9 %.

For the copper pyrithione of which two-thirds was ground with a hammer mill and mixed with one-third that was not ground, oil absorbency and dispersability were 0.39 me /g and 100 µm, respectively, as measured in the same manner as described in Example 1.

### Example 5: Preparation of Fine Powder of Zinc Pyrithione

1000 g of sodium pyrithione, 20% aqueous solution (1.34 mmol of sodium pyrithione) was added to a 2L reactor equipped with a mechanical agitator and warmed to 70 °C with stirring. To the solution was added 190 g of 50 % zinc chloride (0.7 mmol of zinc chloride) over 10 minutes.

After the completion of the addition, the solution was stirred at 70 °C for one hour, dehydrated and then washed with 500 g of distilled water.

The solid thus obtained was filtered through a 50-mesh sieve, and the zinc pyrithione passed through the 50-mesh sieve was analyzed in regard to particle size with a particle size analyzer (Mastersiser 2000 supplied by Malvern Company). The results are presented in Table 1.

The zinc pyrithione thus obtained was 212 g with a yield of 99.5 %, and the purity checked by chemical analysis was 98.8 %.

For the zinc pyrithione, oil absorbency and dispersability were 0.35 mℓ/g and 90 µm, respectively, as measured in the same manner as described in Example 1.

### Example 6: Preparation of Fine Powder of Copper Pyrithione

1000 g of sodium pyrithione, 20% aqueous solution (1.34 mmol of sodium pyrithione) was added to a 2L reactor equipped with a mechanical agitator and warmed to 70 °C with stirring. To the solution was added 186 g of 50 % copper chloride (0.7 mmol of copper chloride) over 60 minutes.

After the completion of the addition, the solution was stirred at 70 °C for one hour, dehydrated and then washed with 500 g of distilled water.

The solid thus obtained was filtered through a 50-mesh sieve, and the copper pyrithione passed through the 50-mesh sieve was analyzed in regard to particle size with a particle size analyzer (Mastersiser 2000 supplied by Malvern Company). The results are presented in Table 1.

The copper pyrithione thus obtained was 209 g with a yield of 98.8 %, and the purity checked by chemical analysis was 99.3 %.

For the copper pyrithione, oil absorbency and dispersability were 0.35 mℓ/g and 80 µm, respectively, as measured in the same manner as described in Example 1.

As can be seen from the results of Examples 1 to 6, the pyrithione metal salts having a defined particle size distribution according to the present invention had an oil absorbency of 0.32 to 0.39 mℓ/g and a dispersability of about 70 to 100 µm, which values are adequate for preparation of an antifouling paint composition.

### Comparative Example 1: Preparation of Fine Powder of Copper Pyrithione

In regrinding the copper pyrithione prepared in Example 2 with an air jet mill, the injection velocity of the copper pyrithione was controlled to obtain a fine powder of copper pyrithione having a smaller average particle size relative to the copper pyrithione of Example 2. The fine powder of copper pyrithione was analyzed in regard to particle size with a particle size analyzer (Mastersiser 2000 supplied by Malvern Company). The results are presented in Table 1.

For the copper pyrithione, oil absorbency and dispersability were 0.30 mℓ/g and 100 µm, respectively, as measured in the same manner as described in Example 1.

### Comparative Example 2: Preparation of Fine Powder of Copper Pyrithione

After the drying step of Example 2, the powder not ground was analyzed in regard to particle size with a particle size analyzer (Mastersiser 2000 supplied by Malvern Company). The results are presented in Table 1.

For the copper pyrithione, oil absorbency and dispersability were 0.40 mℓ/g and 250 µm, respectively, as measured in the same manner as described in Example 1.

**Table 1**

| | | Example | | | | | | Comparative Example | |
|---|---|---|---|---|---|---|---|---|---|
| | | 1 | 2 | 3 | 4 | 5 | 6 | 1 | 2 |
| Particle Size Distribution (µm) | D(0.1) | 1.3 | 1.2 | 1.9 | 1.9 | 1.9 | 1.4 | 1.1 | 2.9 |
| | D(0.2) | 1.9 | 1.9 | 3.4 | 3.2 | 2.2 | 1.6 | 1.3 | 4.2 |
| | D(0.3) | 2.1 | 2.0 | 4.5 | 4.1 | 2.9 | 1.8 | 1.8 | 6.2 |
| | D(0.4) | 2.3 | 2.2 | 5.1 | 4.9 | 3.6 | 2.1 | 1.9 | 7.5 |
| | D(0.5) | 2.6 | 2.3 | 6.3 | 5.8 | 4.1 | 2.6 | 2.2 | 8.3 |
| | D(0.8) | 5.2 | 5.1 | 9.5 | 9.8 | 6.5 | 6.5 | 4.4 | 16.2 |
| | D(0.9) | 5.7 | 5.6 | 15.3 | 14.8 | 7.8 | 7.1 | 4.9 | 29.3 |
| | D(0.95) | 10.7 | 10.3 | 17.8 | 17.4 | 17.3 | 15.4 | 7.2 | 34.9 |
| Average Particle Size (µm)* | 4.8 | 3.7 | 6.9 | 6.3 | 5.3 | 5.0 | 3.4 | 10.3 | |
| Note: * Average particle size is measured with a particle size analyzer. | | | | | | | | | |

In Table 1, for example, the term "D(0,1)" indicates a cumulative volume fraction of smaller-size particles that fall on 10 % of the whole particles ranging from the smallest particles to the largest ones. For Example 1, the particle size of particles of which the cumulative volume fraction is 10 % is 1.3 µm, the particle size for 20 % being 1.9 µm, the particle size for 30 % being 2.1 µm. Hence, the particles having a particle size of less than 2 µm fall on 20 to 30 %, at least 10 % of the whole particles. Considering that D(0.9) and D(0.95) are 5.7 µm and 10.7 µm, respectively, the particles having a particle size of greater than 10 µm fall on 5 to 10 %, at least 5 % of the whole particles.

For Example 2, the particles having a particle size of less than 2 µm fall on 20 to 30 %, at least 10 % of the whole particles, and those having a particle size of greater than 10 µm fall on 5 to 10 %, at least 5 % of the whole particles. As for Example 3, the particles having a particle size of less than 2 µm belong to at least 10 % of the whole particles, and those having a particle size of greater than 10 µm belong to 10 to 20 % of the whole particles. For Example 4, the particles having a particle size of less than 2 µm fall on at least 10 % of the whole particles, and those having a particle size of greater than 10 µm fall on 20 to 30 % of the whole particles. As for Example 5, the particles having a particle size of less than 2 µm belong to at least 10 % of the whole particles, and those having a particle size of greater than 10 µm belong to 5 to 10 % of the whole particles. For Example 6, the particles having a particle size of less than 2 µm fall on at least 30 to 50 %, at least 10 % of the whole particles, and those having a particle size of greater than 10 µm fall on 5 to 10 % of the whole particles.

For Comparative Example 1, however, the particles having a particle size of less than 2 µm fall on 30 to 50 %, at least 10 % of the whole particles, and those having a particle size of greater than 10 µm fall on less than 5 % of the whole particles. As for Comparative Example 2, the particles having a particle size of less than 10 µm belong to 20 to 50 %, at least 5 % of the whole particles, and those having a particle size of less than 2 µm belong to less than 10 % of the whole particles.

The pyrithione metal salts having the defined particle size distribution according to the present invention are compared with the pyrithione metal salts according to the Comparative Examples in regard to antifouling characteristics when used for a paint composition.

### <Preparation of Paint Composition>

### Example 7

72 g of acrylic acid, 500 g of methyl methacrylate and 428 g of ethyl methacrylate were copolymerized to obtain a copolymer having a number average molecular weight of about 10,000, 50 % butyl acetate solution. To the resultant solution was added 80 g of zinc oxide, 50 g of butanol and 10 g of water and stood for reaction at 120 °C for 10 hours to prepare a 50%-solid clear resin solution.

To 100 g of the resin solution thus obtained were added 5 g of chlorinated paraffin, 20 g of the zinc pyrithione of Example 1, 40 g of copper suboxide, 4 g of bengala, 1 g of aerogel #2 (silica powder, supplied by Degussa, Germany) and 22 g of xylene for dispersion to prepare an antifouling paint composition.

The antifouling paint composition thus obtained was coated and dried into thickness of 100 µm on a 100*300*3.5mm test sheet with an anticorrosive coating of vinyl tar paint, and checked in regard to its film formation condition by macroscopic observation. The results are presented in Table 2.

On the other hand, a dry glass panel was immersed 1 m below sea level on a reef on a beach in Geojedo island, Kyungnam to check the attachment of marine life. The results are presented in Table 2.

### Examples 8 to 12

Paint compositions were prepared in the same manner as described in Example 7, excepting that the pyrithione metal salts powders of Examples 2 to 6 rather than that of Example 1 were used as an antifouling effective component.

The paint compositions thus obtained were analyzed in regard to film formation condition and antifouling performance in the same manner as described in Example 1. The results are presented in Table 2.

### Comparative Examples 3 and 4

Paint compositions were prepared in the same manner as described in Example 7, excepting that the pyrithione metal salts powders of Comparative Examples 1 and 2 rather than that of Example 1 were used as an antifouling effective component.

The paint compositions thus obtained were analyzed in regard to film formation condition and antifouling performance in the same manner as described in Example 1. The results are presented in Table 2.

**Table 2**

| | | Example | | | | | | Comparative Example | |
|---|---|---|---|---|---|---|---|---|---|
| | | 7 | 8 | 9 | 10 | 11 | 12 | 3 | 4 |
| Type of Pyrithione metal salts | | 1 | 2 | 3 | 4 | 5 | 6 | 1 | 2 |

| Film Formation Condition | | Good | Good | Good | Good | Good | Good | Good | Bad |
|---|---|---|---|---|---|---|---|---|---|
| Attachment Rate of Marine Life | 1 month | 0% | 0% | 0% | 0% | 0% | 0% | 0% | 0% |
| | 2 months | 0% | 0% | 0% | 0% | 0% | 0% | 0% | 5% |
| | 3 months | 5% | 5% | 2~3% | 2% | 0% | 0% | 10% | 15% |
| | 6 months | 20% | 15% | 30% | 30% | 0% | 5% | 50% | 45% |
| | 12 months | 30% | 35% | 60% | 70% | 10% | 10% | 90% | 65% |

As can be seen from the results of Table2, the paint composition containing the pyrithione metal salts of the present invention is excellent in film formation condition and antifouling durability. As for the Comparative Example 3, the use of a pyrithione metal salts having a good dispersability provides a good film formation condition, but the paint composition in which the particles having a particle size of greater than 10 µm fall on less than 5 % of the whole particles has a deterioration of antifouling durability. For the Comparative Example 4, the dispersability deteriorates and the paint composition contains a pyrithione metal salts of which the particles having a particle size of less than 2 µm are less than 10 % of the whole particles, to exhibit poor film formation condition with a deterioration of initial antifouling performance and antifouling durability.

As described above, the pyrithione metal salts of the present invention that contains a defined content of fine particles having a very small particle size in consideration of the initial antifouling performance and a defined content of particles having a particle size of greater than 10.0 µm guarantees an oil absorbency adequate for an antifouling paint and good dispersability in preparing a paint composition. The paint composition containing the pyrithione metal salts provides a good film formation condition with enhanced antifouling durability to guarantee an antifouling performance for a long-term use.

## Claims

1. A pyrithione metal salts comprising, in the aspect of a particle size distribution, 10 to 40 wt.% of particles having a particle size of less than 2 µm, and 5 to 20 wt.% of particles having a particle size of greater than 10.0 µm.

2. The pyrithione metal salts as claimed in claim 1, wherein the pyrithione metal salts comprises at least one compound represented by the following formula 1: 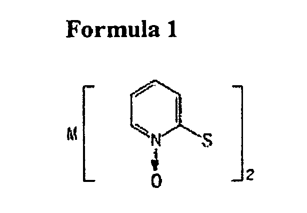 Wherein M is Zn, Cu, Mg, Ba or Sr.

3. A paint composition containing a pyrithione metal salts as an antifouling effective component, the pyrithione metal salts comprising, in the aspect of particle size distribution, 10 to 40 wt.% of particles having a particle size of less than 2 µm, and 5 to 20 wt.% of particles having a particle size of greater than 10.0 µm.

4. The paint composition as claimed in claim 3, wherein the pyrithione metal salts comprises at least one compound represented by the following formula 1: 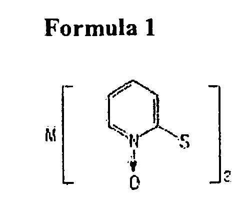 Wherein M is Zn, Cu, Mg, Ba or Sr.

5. The paint composition as claimed in claim 3 or 4, wherein the pyrithione metal salts is used in an amount of 1 to 20 wt.% in the total weight of the paint composition.

## Patentansprüche

1. Pyrithionmetallsalze, umfassend, unter dem Gesichtspunkt der Teilchengrö-ßenverteilung, 10 bis 40 Gew.-% Teilchen mit einer Teilchengröße von weniger als 2 µm und 5 bis 20 Gew.-% Teilchen mit einer Teilchengröße von größer als 10,0 µm.

2. Pyrithionmetallsalze nach Anspruch 1, wobei die Pyrithionmetallsalze mindestens eine Verbindung der folgenden Formel 1 umfassen: 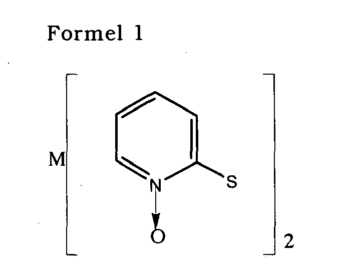 worin M die Bedeutung Zn, Cu, Mg, Ba oder Sr hat.

3. Farbzusammensetzung, enthaltend ein Pyrithionmetallsalz als wirksame Antifouling-Komponente, wobei das Pyrithionmetallsalz, unter dem Gesichtspunkt der Teilchengrößenverteilung, 10 bis 40 Gew.-% Teilchen mit einer Teilchengröße von weniger als 2 µm und 5 bis 20 Gew.-% Teilchen mit einer Teilchengröße von größer als 10,0 µm umfasst.

4. Farbzusammensetzung nach Anspruch 3, wobei die Pyrithionmetallsalze mindestens eine Verbindung der folgenden Formel 1 umfassen: 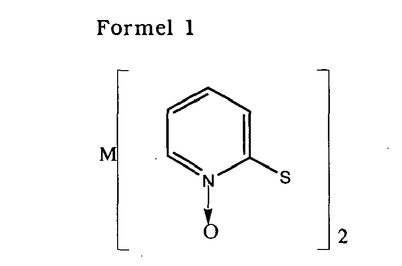 worin M die Bedeutung Zn, Cu, Mg, Ba oder Sr hat.

5. Farbzusammensetzung nach Anspruch 3 oder 4, wobei das Pyrithionmetallsalz in einer Menge von 1 bis 20 Gew.-%, bezogen auf das Gesamtgewicht der Farbzusammensetzung, verwendet wird.

## Revendications

1. Sels métalliques de pyrithione comprenant sous l'aspect de la distribution de taille de particule, 10 à 40% un poids de particules ayant une taille de particule inférieure à 2 µm et 5 à 20% en poids de particules ayant une taille de particule supérieure à 10,0 µm.

2. Sels métalliques de pyrithione selon la revendication 1, dans lesquels les sels métalliques de pyrithione comprennent au moins un composé représenté par la formule 1 suivante : 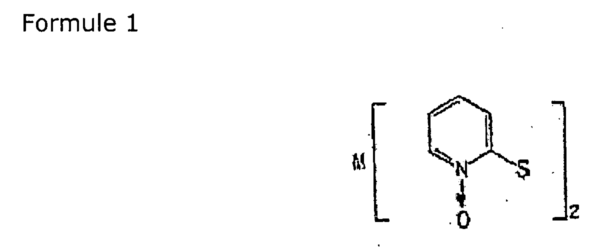 dans laquelle M est du Zn, du Cu, du Mg, du Ba ou du Sr.

3. Une composition de peinture contenant des sels métalliques de pyrithione en tant que composé actif imputrescible, les sels métalliques de pyrithione comprenant sous l'aspect de la distribution de taille de particule, 10 à 40% un poids de particules ayant une taille de particule inférieure à 2 µm et 5 à 20% en poids de particules ayant une taille de particule supérieure à 10,0 µm.

4. La composition de peinture selon la revendication 3, dans laquelle les sels métalliques de pyrithione comprennent au moins un composé représenté par la formule 1 suivante : 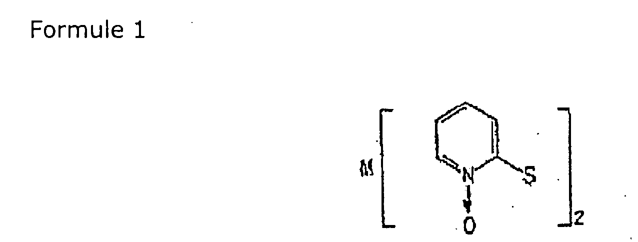 dans laquelle M est du Zn, du Cu, du Mg, du Ba ou du Sr.

5. La composition de peinture selon la revendication 3 ou 4, dans laquelle les sels métalliques de pyrithione sont utilisés dans une quantité comprise entre 1 et 20% en poids du poids total de la composition de peinture.
